# FASCICULE DE BREVET EUROPEEN

(11) **EP 1 250 124 B1**
(45) Date de publication et mention de la délivrance du brevet: **23.11.2005**
(21) Numéro de dépôt: 01907625.6
(22) Date de dépôt: 18.01.2001
(51) Int. Cl.: A61K 7/48

(54) **COMPOSITION COSMETIQUE AMINCISSANTE COMPRENANT UN EXTRAIT DE PLANTE CONTENANT UN PEPTIDE NATRIURETIQUE**
KOSMETISCHES SCHLANKHEIDSMITTEL ENTHALTEND EINEN PFLANZEN EXTRAKTEN MIT EINEM NATRIURETISCHEM PEPTID
SLIMMING COSMETIC COMPOSITION COMPRISING AS ACTIVE AGENT A PLANT EXTRACT CONTAINING A PLANT NATRIURETIC PEPTIDE (PNP)

(30) Priorité: 28.01.2000 FR 0001151
(43) Date de publication de la demande: 23.10.2002
(73) Titulaire: Laboratoires Clarins, 92200 Neuilly sur Seine (FR)
(72) Inventeur: COURTIN, Olivier, F-92200 Boulogne sur Seine (FR)
(74) Mandataire: Breese, Pierre
(86) Numéro de dépôt international: PCT/FR2001/000160
(87) Numéro de publication internationale: WO 2001/054659

(56) Documents cités:
- FR-A- 2 554 344
- FR-A- 2 729 856
- FR-A- 2 748 659
- FR-A- 2 758 724
- FR-A- 2 775 595
- US-A- 5 194 259
- DATABASE WPI Week 199747 Derwent Publications Ltd., London, GB; AN 1997-508766 XP002150087 & JP 09 241127 A (NONOGAWA SHOJI KK)
- PATENT ABSTRACTS OF JAPAN vol. 017, no. 025 (C-1017) & JP 04 247012 A (MIKIMOTO SEIYAKU KK)
- DATABASE MEDLINE [en ligne] YANG ET AL.: "ATRIAL NATRIURETIC-LIKE PEPTIDE AND ITS PROHORMONE WITHIN METASEQUOIA" retrieved from STN Database accession no. 1999333817 XP002150084
- DATABASE CAPLUS [en ligne] VESELY ET AL.: "ATRIAL NATRIURETIC PEPTIDE HORMONAL SYSTEM IN PLANTS" retrieved from STN Database accession no. 1991:579394 XP002150085
- DATABASE CAPLUS [en ligne] PHARMAWATI ET AL.: "AN IMMUNOAFFINITY PURIFIED PLANT NATRIURETIC PEPTIDE ANALOG MODULATES cGMP LEVELS IN THE ZEA MAYS ROOT STELE" retrieved from STN Database accession no. 1998:554123 XP002150086
- DATABASE WPI Week 200030 Derwent Publications Ltd., London, GB; AN 2000-342400 XP002172872 & JP 2000 063237 A (KANEBO)

## Description

La présente invention concerne l'utilisation d'ANP et plus particulièrement d'un extrait d'origine végétale contenant de l'ANP, ci-après aussi désigné « PNP » ou « peptide natriurétique de plante, pour favoriser la lipolyse et remarquable pour la préparation de compositions cosmétiques amincissantes.

L'hypoderme ou tissu adipeux constitue une couche de graisse de réserve qui enveloppe complètement le corps, mais dont l'épaisseur est variable. Chez l'homme, il est prépondérant au-dessus de la ceinture, principalement au niveau de l'abdomen, chez la femme, il se concentre au-dessous de la ceinture, dans la zone des fesses, cuisses et de la partie basse du de l'abdomen. Le tissu adipeux représente 15 à 20% du poids corporel ce qui correspond à plus de 50 milliards de cellules adipeuses ou adipocytes. L'adipocyte est capable de stocker des lipides sous forme de triglycérides (TG) ou inversement de mobiliser rapidement les triglycérides qui sont les sources énergétiques majeures de l'organisme. La régulation de ces deux processus dépend de facteurs hormonaux et de signaux cellulaires. En période de jeûne, les adipocytes fournissent l'énergie nécessaire au fonctionnement de l'organisme, selon un processus connu sous le nom de lipolyse, durant lequel les triglycérides sont hydrolysés en acides gras et en glycérol.

La lipolyse est sous le contrôle d'une enzyme principale désignée triglycéride-lipase (TG-lipase). La TG-lipase est directement stimulée par l'AMP cyclique (AMPc). La concentration en AMPc est sous la dépendance d'une part de l'adénylate cyclase dont l'activation conduit à l'AMPc, et d'autre part, de l'AMPc phosphodiestérase dont l'activation est responsable de la dégradation de l'AMPc. L'activité de l'adénylate cyclase dans les adipocytes est régulée par les catécholamines et notamment l'adrénaline qui se fixe sur deux types de récepteurs :
- les récepteurs bêta adrénergiques, qui activent l'adénylate cyclase et donc stimule la lipolyse via l'activation de l'AMPc,
- les récepteurs alpha adrénergique qui inhibent l'adénylate cyclase et donc également la lipolyse.

Il a donc été proposé d'utiliser dans l'art antérieur de nombreuses substances susceptibles d'interagir avec les récepteurs alpha et/ou bêta adrénergique pour stimuler la lipolyse. Ainsi, la caféine est très utilisée en cosmétique dans des compositions notamment des crèmes amincissantes.

On connaît par ailleurs les protéines G découvertes par Gilman et Rodbell, qui sont impliquées dans le système de transport d'hormone extracellulaire vers l'AMPc. Les protéines G lie la GTP et active plusieurs enzymes dont l'adénylate cyclase qui, comme indiqué précédemment, favorise la formation d'APMc, lequel stimule la TG-lipase et donc la lipolyse. Il a ainsi été proposé dans l'art antérieur d'utiliser des substances capables d'activer les protéines G pour augmenter la lipolyse. Dans le domaine cosmétique, on connaît par exemple un extrait d'Atractyldes Lancea (racine), qui est une plante d'origine chinoise connue sous le nom de Cang Zhu, utilisé en association avec la caféine pour la préparation de compositions amincissantes.

On connaît encore le rôle de la guanylate cyclase qui transforme le GTP en GMPc lequel stimule les lipases, notamment la TG-lipase, et donc favorise la lipolyse.

L'ANP ou « peptide natriurétique atrial » est libéré par les cellules de l'atrium du coeur lorsque celui-ci est distendu par une augmentation du volume sanguin. Le sang transporte l'ANP jusqu'aux reins ou il active la guanylate cyclase des cellules des tubes collecteurs, ce qui favorise la formation de GMPc et augmente l'excrétion rénale de sodium et par conséquence augmente l'excrétion d'eau ce qui entraîne une baisse du volume sanguin.

On vient maintenant de mettre en évidence la présence de récepteurs à l'ANP au niveau des adipocytes, lequel permet, selon les mécanismes décrits ci-dessus, de stimuler la guanylate cyclase et donc la formation de GMPc qui via la TG-lipase favorise la lipolyse.

La Demanderesse a maintenant mis à profit le rôle de l'ANP pour concevoir de nouvelles compositions favorisant la lipolyse comprenant en tant qu'agent actif de l'ANP et plus particulièrement un extrait de plante contenant de l'ANP utile dans le domaine cosmétique pour ses propriétés amincissantes.

Or, dans le domaine cosmétique, on préfère tout particulièrement utiliser des extraits végétaux. Ainsi, l'invention concerne l'utilisation d'un extrait de plante contenant de l'ANP comme agent cosmétique stimulant la lipolyse pour la préparation d'une composition amincissante.

Il a en effet été décrit dans le règne végétal de nombreux ANP, désigné aussi ANP «like», et dénommé également ci-après PNP pour « peptide natriurétique de plante » présent dans les tiges, les feuilles, les pétales de plantes (David L. Vesely et Amy T. Giordano, Biochemical and Biophysical Research communications, August 30, 1991, Vol. 179 No 1, pp 6.95-700 ; J.O. Adeboye et al., 1997, Phytotherapy Research, Vol No 11, pp 454-456, Pulock K ; Mukherjee et al., Phytotherapy Research, 1996, Vol No 10, pp 424-425) comme Florida beauty, budhist pine, boston fer, rose, géranium, ressurection plant ou club moss, moses in the cradle, florida coontie. Il s'agit aussi principalement des plantes suivantes : Vermonia Cinerea, Nelumbo nucifera (lotus sacré), Allium sativum (aïl), Dracena godseffiana, Metasequoia glyptostroboides, Hedera helix (lierre).

Ainsi, une composition cosmétique selon l'invention comprend au moins extrait des plantes choisies parmi dans le groupe comprenant : Geranium Robertianum, Vermonia Cinerea, , Allium sativum, Dracena godseffiana, Metasequoia glyptostroboides, .

Les compositions selon l'invention trouvent donc leur application dans le traitement de l'obésité et plus particulièrement comme composition amincissante dans le domaine cosmétique.

En effet, le corps, plus particulièrement de la femme, subi au cours du temps des modifications inesthétiques notamment du tissu adipeux liées à plusieurs facteurs qui peuvent être différents selon les individus. Il s'agit, bien entendu, du vieillissement, mais aussi, de facteurs alimentaires ou pathologiques. On a donc proposé dans l'art antérieur de nombreuses compositions cosmétiques amincissantes à base d'extraits de plantes. On peut citer plus particulièrement, les compositions cosmétiques amincissantes décrites dans le brevet français de la Demanderesse publié sous le No. 2 729 856. Ce brevet propose des compositions cosmétiques amincissantes capables par le biais de substances organiques solubles, ou enzymes, d'affiner et de préparer la peau à recevoir des principes actifs dont les effets sont potentialisés pour favoriser l'élimination des lipides et prévenir le stockage de ces lipides.

On peut également citer les compositions cosmétiques amincissantes décrites dans le brevet américain US 5,194,259. Ce brevet décrit des compositions cosmétiques amincissantes comprenant au moins un alpha-2 bloqueur issu du Ginko boloba et au moins un alpha-2 bloqueur issu soit du lierre (Hedera helix), soit de l'escine. Les extrait de plante utilisées dans ces compositions et notamment l'extrait de Hedera helix est riche en saponines obtenu par élimination de constituants tels qu'entre autres les protéines. Ces compositions sont donc dépourvues de PNP.

Le brevet français 2 554 344 décrit quant à lui une composition cosmétique amincissante et anti-cellulite contenant une base purique, un extrait de Hedera helix et un extrait de Ruscus aculeatus. Dans cette composition cosmétique amincissante l'agent agissant sur le processus de lipolyse est une base purique, les extrait de Hedera helix et de Ruscus aculeatus n'étant quant à eux que préconisé pour leurs propriétés vaso-constrictrices, anti-exsudatives et capillaro-protectrices. L'extrait de Hedera helix utilisé est à fortiori avantageusement constitué par un extrait hydroalcoolique titré en hédérine et en hédéragénine.

D'autres compositions cosmétiques contenant des extraits de plante en tant qu'agents actifs ont été décrites dans l'art antérieur. Parmi ces dernières nous pouvons cité le la demande de brevet japonaise 04247012 qui décrit un produit cosmétique contenant en tant qu'agent actif un extrait de feuille de Nelumbo nucifera. Dans cette composition cosmétique l'extrait de feuille de Nelumbo nucifera est utilisé pour ses propriétés embellissantes, blanchissantes ainsi que pour ses effets de prévention contre la rugosité de la peau. La brevet japonaise 04247012 ne décrit ni ne suggère d'utiliser un extrait de feuilles de Nelumbo nucifera dans une composition cosmétique amincissante pour favoriser la lipolyse.

Une composition cosmétique amincissante selon l'invention est donc caractérisée en ce qu'elle comprend en tant qu'agent actif du PNP, de préférence sous la forme d'un extrait de plante contenant du PNP à l'exception d'un extrait des plantes Hedera helix et Nelumbo nucifera, en tant que facteur stimulant la lipolyse et destiné à lutter contre une augmentation du volume des adipocytes.

Outre l'extrait de plante contenant du PNP, les compositions de l'invention peuvent comprendre d'autres actifs utiles pour inhiber la lipogénèse ou favoriser la lipolyse, ou encore pour affiner, raffermir, lifter la surface cutanée ou l'hydrater. On peut citer plus particulièrement un extrait d'une plante riche en hydroxycitrate, comme un extrait d'écorce du fruit de Garcinia Cambodgia. Garcinia Cambodgia est une plante originaire des Indes, riche en hydroxycitrate, qui est un inhibiteur de la lipogénèse donc du stockage des graisses. Un tel extrait d'écorce du fruit de Garcinia Cambodgia est commercialisé, sous la dénomination Garcinol, en association avec un biofiltrat de Candida Lipolytica. Le biofiltrat de *Candida Lipolytica* est obtenu par fermentation de cette souche sur milieu spécifique, il permet de stimuler la lipolyse, plus particulièrement l'hydrolyse des triglycérides, et donc favorise le déstockage des graisses.

Outre, le Garcinol, les compositions cosmétiques amincissantes selon l'invention peuvent comprendre de la caféine, qui présente une action lipolytique et renforce celle du GARCINOL, ou encore un ou plusieurs des extraits suivants :
- Un extrait de Fragon ou Petit Houx ou encore Ruscus, qui est riche en matières minérales, comme le calcium, le potassium, et en saponosides et sapogénines. La présence de saponosides confère à cet extrait des propriétés :
   . anti-inflammatoire, calmantes et apaisantes,
   . éclaircissante efficace pour la protection contre les rougeurs,
   . qui favorise la microcirculation et donc améliore la tonicité des capillaires,
   . décongestionnante cutanée.
- Un ou plusieurs dérivés de silicium, comme le SILANOL, qui présente les propriétés suivantes :
   . hydratante et permet de régulariser la concentration hydrique,
   . de prévention et de régénération, notamment une activité anti-radicalaire qui permet une réorganisation des lipides membranaires en rendant la membrane plus résistante à l'attaque des radicaux libres.
   . anti-inflammatoire, qui diminue la vasodilatation et annule l'action de l'acide arachidonique.
- Un extrait de Marron d'Inde, qui présente des propriétés :
   . vitaminiques P, qui permettent d'augmenter la résistance et de diminuer la perméabilité des capillaires sanguins (vasoprotecteur),
   . décongestionnantes et adoucissantes sur l'inflammantion, car les dérivés saponosiques du marron d'inde permettent de traiter l'effet "peau d'orange".
- Un extrait de Ginkgo Biloba, dont les feuilles sèches sont utilisées en médecine comme tonique veineux du fait de leur teneur en flavoides.
- Un extrait de vigne rouge. Les feuilles de vigne rouge sont riches en flavonoïdes et permettent de renforcer les parois des vaisseaux et le tonus veineux. Un tel extrait agit donc sur la microcirculation.
- Un extrait d'algues rouges, qui permet de protéger le tissu conjonctif et d'atténuer la fragilité des capilaires.

La proportion de l'extrait de plante contenant de l'ANP dans les compositions selon l'invention est comprise entre 0,5 à 10 %, de préférence de l'ordre de 1 à 5 %

En outre, les compositions cosmétiques amincissante selon l'invention peuvent contenir un ou plusieurs agents susceptible de conférer à la composition de l'invention, des propriétés apaisantes ou des propriétés tonifiantes. Ainsi, l'invention envisage des compositions cosmétiques amincissantes et apaisantes, utiles comme après-soleil, après-rasage, spéciales peaux sensibles, etc..., dans lesquels l'actif apaisant est par exemple, l'allantoïne, l'acide 18 bêta glycyrrhétinique, du bisabolol, un extrait de plantes apaisantes comme le tilleul ou la camomille.

L'invention envisage donc aussi des compositions cosmétiques amincissantes et tonifiantes, dans lesquels l'actif tonifiant est par exemple le ginseng, l'éleutérocoque, le citron, l'orange, le rathania, etc....

Les compositions de l'invention peuvent se présenter sous toute forme connue de l'homme du métier dans le domaine de la cosmétique, comme par exemple, une crème, un lait, une lotion, un gel, un masque, etc..., sans aucune restriction galénique particulière autre que celles pour l'application sur la peau.

## Revendications

1. Composition cosmétique amincissante, **caractérisée en ce qu'**elle comprend en tant qu'agent actif du PNP, de préférence sous la forme d'un extrait de plante contenant du PNP, à l'exception d'un extrait de la plante Hedera helix et d'un extrait de la plante Nelumbo nucifera.

2. Composition cosmétique selon la revendication 1, **caractérisée en ce que** ledit extrait de plante contenant du PNP est l'un au moins des extraits de plantes choisies dans le groupe comprenant : Geranium Robertianum, Vermonia Cinerea, Allium sativum, Dracena godseffiana et Metasequoia glyptostroboides.

3. Composition cosmétique selon l'une des revendications 1 ou 2, **caractérisée en ce qu'**elle comprend en outre au moins un agent actif utile pour inhiber la lipogenèse ou favoriser la lipolyse ou encore pour affiner, raffermir, lifter la surface cutanée ou l'hydrater.

4. Composition cosmétique selon la revendication 3, **caractérisée en ce qu'**elle comprend un extrait d'une plante riche en hydroxycitrate, comme un extrait d'écorce du fruit de Garcinia Cambodgia.

5. Composition cosmétique selon l'une des revendications 3 ou 4, **caractérisée en ce qu'**elle comprend de la caféine.

6. Composition cosmétique selon l'une quelconque des revendications 3 à 5, **caractérisée en ce qu'**elle comprend un ou plusieurs des composants choisis dans le groupe suivant:
- un extrait de Fragon,
- un ou plusieurs dérivés de silicium,
- un extrait de Marron d'Inde,
- un extrait de Ginkgo Biloba,
- un extrait de vigne rouge,
- un extrait d'algues rouges.

7. Composition cosmétique selon l'une quelconque des revendications 1 à 6, **caractérisée en ce que** la proportion de l'extrait de plante contenant du PNP dans ladite composition est comprise entre 0,5 à 10 %, de préférence de l'ordre de 1 à 5 %.

8. Utilisation d'un extrait de plante contenant du PNP comme agent cosmétique stimulant la lipolyse pour la préparation d'une composition amincissante.

## Patentansprüche

1. Schlank machende kosmetische Zusammensetzung, charakterisiert **dadurch** dass sie als aktives Mittel PNP enthält, vorzugsweise in Form eines Pflanzenextrakts enthaltend PNP, ausgenommen ein Extrakt der Pflanze Hedera helix und eines Extrakts der Pflanze Nelumbo nucifera.

2. Kosmetische Zusammensetzung nach Anspruch 1, charakterisiert **dadurch** dass besagtes Pflanzenextrakt enthaltend PNP ist wenigstens eines der Pflanzenextrakte ausgewählt aus der Gruppe enthaltend: Geranium Robertianum, Vermonia Cinerea, Allium sativum, Dracena godseffinana und Metasequoia glyptostroboides.

3. Kosmetische Zusammensetzung nach einem der Ansprüche 1 oder 2, charakterisiert **dadurch** dass sie außerdem enthält ein aktives Mittel nützlich dazu die Lipogenese zu blockieren oder die Lipolyse zu fördern oder dazu die Hautoberfläche zu verfeinern, zu festigen, zu liften oder zu hydrieren.

4. Kosmetische Zusammensetzung nach Anspruch 3, charakterisiert **dadurch** dass sie enthält ein Extrakt einer Pflanze reich an Hydroxycitrat, wie ein Extrakt der Rinde der Frucht von Garcinia Cambodgia.

5. Kosmetische Zusammensetzung nach einem der Ansprüche 3 oder 4, charakterisiert **dadurch** dass sie Koffein enthält.

6. Kosmetische Zusammensetzung nach einem der Ansprüche 3 bis 5, charakterisiert **dadurch** dass sie enthält einen oder mehrere Bestandteile ausgewählt aus der folgenden Gruppe:
- ein Extrakt von Fragon
- eines oder mehrere Derivate von Silizium
- ein Extrakt von indischen Kastanien
- ein Extrakt von Ginkgo Biloba
- ein Extrakt von roter Rebe
- ein Extrakt von roten Algen

7. Kosmetische Zusammensetzung nach einem der Ansprüche 1 bis 6, charakterisiert **dadurch** dass die Proportion von PNP enthaltendem Pflanzenextrakt in besagter Zusammensetzung zwischen 0,5 und 10 % liegt, vorzugsweise in der Größenordnung zwischen 1 und 5 %.

8. Gebrauch eines Pflanzenextrakts enthaltend PNP als kosmetisches Mittel stimulierend die Lipolyse für die Vorbereitung einer schlank machenden Zusammensetzung.

## Claims

1. Cosmetic slimming composition, **characterised in that** it comprises PNP as an active agent, preferentially in the form of a plant extract containing PNP, with the exception of a Hedera helix plant extract, and of a Nelumbo nucifera plant extract.

2. Cosmetic composition according to claim 1, **characterised in that** said plant extract containing PNP is at least one of the plant extracts selected from the group comprising: Geranium Robertanium, Vermonia Cinerea, Allium sativum, Dracena godseffiana and Metasequoia glyptostroboides.

3. Cosmetic composition according to any one of claims 1 or 2, **characterised in that** it further comprises at least one active agent necessary to inhibit lipogenesis or promote lipolysis or to slim, firm, lift or moisturize the skin surface.

4. Cosmetic composition according to claim 3, **characterised in** it comprises an extract of a plant rich in hydroxycitrate, such as a Garcinia Cambodgia fruit skin extract.

5. Cosmetic composition according to any one of claims 3 or 4, **characterised in that** it comprises caffeine.

6. Cosmetic composition according to any one of claims 3 to 5, **characterised in that** it comprises one or more of the constituents selected from the following group:
- a Ruscus extract,
- one or more silicon derivatives,
- a Horse Chestnut extract,
- a Gingko Biloba extract,
- a red vine extract,
- a red algae extract.

7. Cosmetic composition according to any one of claims 1 to 6, **characterised in that** the proportion of the plant extract containing PNP in said formulation is between 0.5 and 10%, preferentially of the order of 1 to 5%.

8. Use of a plant extract containing PNP as a cosmetic agent stimulating lipolysis for the preparation of a slimming composition.
